# EUROPEAN PATENT SPECIFICATION

(11) **EP 1 641 772 B1**
(45) Date of publication and mention of the grant of the patent: **31.12.2008**
(21) Application number: 04740420.7
(22) Date of filing: 29.06.2004
(51) Int. Cl.: C07D 311/72, B01J 23/887

(54) **MANUFACTURE OF TOCOPHEROLS USING A BISMUTH CATALYST**
HERSTELLUNG VON TOKOPHEROLEN UNTER VERWENDUNG EINES WISMUT KATALYSATORS
PRODUCTION DE TOCOPHEROLS METTANT EN OEUVRE UN CATALYSEUR A BASE DE BISMUTH

(30) Priority: 08.07.2003 EP 03015368
(43) Date of publication of application: 05.04.2006
(73) Proprietor: DSM IP Assets B.V., 6411 TE Heerlen (NL)
(72) Inventor: BONRATH, Werner, 79115 Freiburg (DE); GIRAUDI, Lisa, F-68330 Huningue (FR)
(74) Representative: Steck, Melanie
(86) International application number: PCT/EP2004/007026
(87) International publication number: WO 2005/005407

(56) References cited:
- EP-A- 0 658 552
- EP-A- 1 000 940
- WO-A-01/64695

## Description

The present invention is concerned with a novel process for the manufacture of tocols and tocopherols, preferably α-tocopherol (TCP), or its acetate α-tocopheryl acetate (TCPA) by the reaction of a hydroquinone comprising 0 to 3 methyl groups, preferably by the reaction of 2,3,5-trimethylhydroquinone (TMHQ), and 2,3,6-trimethylhydroquinone-1-acetate (TMHQA), respectively, with phytol (PH) or a phytol derivative, e.g. isophytol (IP) or a(n) (iso)phytyl compound, in the presence of bismuth trifluoromethanesulphonate, Bi(OSO₂CF₃)₃, as the catalyst in an organic solvent.

As is known, (all-*rac*)-α-tocopherol (or as it has mostly been denoted in the prior art, "d,1-α-tocopherol") is a diastereoisomeric mixture of 2,5,7,8-tetramethyl-2-(4',8',12'-trimethyl-tridecyl)-6-chromanol (α-tocopherol), which is the biologically most active and industrially most important member of the vitamin E group.

Many processes for the manufacture of "d,1-α-tocopherol" (referred to as such in the literature reviewed hereinafter) and its acetate by the reaction of TMHQ/TMHQA with IP or PH in the presence of a catalyst or catalyst system and in a solvent or solvent system are described in the following selected literature.

In Applied Catalysis A: General 202 (2000), pages 117 to 120 "microencapsulated" (MC) catalysts, MC-(F₃CSO₂)₂NH and MC-Sc(OSO₂CF₃)₃, are used for the synthesis of TCP starting from TMHQ and IP. Unfavourably the MC-catalysts cannot be recycled and loose their activity after a single employment.

Journal of Catalysis 182, 282-284 (1999) describes the use of heterogeneous solid acid catalysts such as Nafion^{®} NR 50, a copolymer of tetraffuoroethene and a perfluoro-sulfonylether, or Amberlyst^{®} 15, a strongly acidic cation exchange resin with SO₃H functional groups, for the TCP synthesis. These catalysts however are rather expensive.

In the processes of DE-OS 2160 103 as well as US 3,789,086 compounds of the following formula 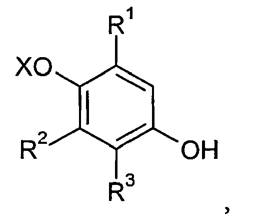 wherein X is hydrogen, alkanoyl or aroyl, and R¹, R² and R³ are individually hydrogen or methyl, are reacted with compounds of the following formulae 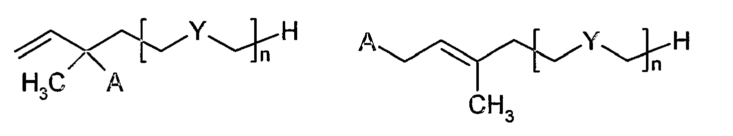 wherein Y is -CH₂-CH(CH₃)- or -CH=C(CH₃)- and A is halogen, hydroxy, etherified hydroxy or esterified hydroxy in the presence of HCl and Fe and/or FeCl₂ as the catalyst to obtain tocols and tocopherols such as α-tocopherol.

TCP e.g. can be converted into its acetate, succinate, and further known application forms by standard methods, e. g. as described in Ullmann's Encyclopedia of Industrial Chemistry, Vol. A27, 5th edition, pages 484 to 485, VCH Verlagsgesellschaft mbH, D-69451 Weinheim, 1996. In contrast to TCP which is labile against oxidative conditions, the esters are more stable and more convenient to handle.

The object of the present invention is to provide a process for the manufacture of tocols and tocopherols, especially α-tocopherol, and α-tocopherol acetate by using a catalyst and a solvent wherein the catalyst used has no, or at least a much reduced, corrosive action, is non-toxic, does not contaminate the environment, and catalyzes the desired reaction as selectively as possible and in high yields. Furthermore, the catalyst should display its activity in small, really catalytic, amounts and should be readily separable and re-usable several times.

The object of the present invention is achieved
by the reaction of a compound a) of the formula II with X¹, X² and X³ being independently from each other hydrogen or methyl and R² being hydrogen or acetyl with the proviso that R² is only acetyl when X¹, X² and X³ are all methyl (= TMHQA), 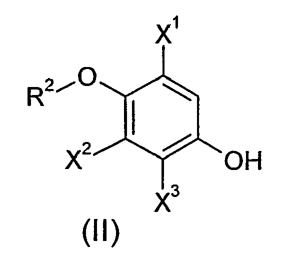 i.e. a hydroquinone with 0 to 3 methyl groups or trimethylhydroquinone acetate namely TMHQ (formula II with X¹ = X² = X³ = methyl and R² = hydrogen), TMHQA (formula II with X¹ = X² = X³ = methyl and R² = acetyl), 2,3-dimethylhydroquinone, 2,5-dimethylhydroquinone, 2,6-dimethylhydroquinone, 2-methylhydroquinone or hydroquinone,
preferably by the reaction of TMHQ or TMHQA, with
a compound b) selected from the group consisting of PH (formula IV with R³ = OH), IP (formula III with R³ = OH), and (iso)phytol derivatives represented by the following formulae III and IV with R³ = C₂₋₅-alkanoyloxy (C₁₋₄-alkyl-C(O)O), benzoyloxy (C₆H₅(CO)O), methanesulfonyloxy (= mesyloxy; H₃C(SO₂)O), benzenesulfonyloxy (C₆H₅(SO₂)O) or toluenesulfonyloxy (= tosyloxy; H₃C-C₆H₄(SO₂)O), 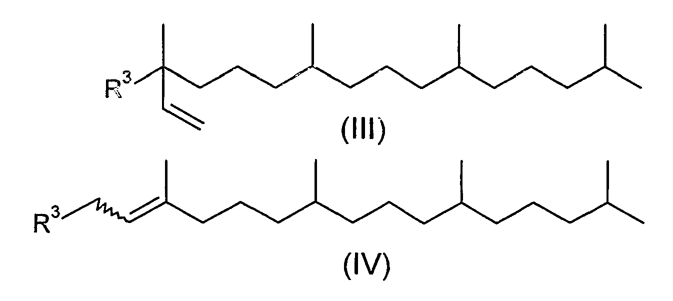 preferably with a compound b) selected from the group consisting of PH, IP and (iso)-phytol derivatives of the formulae III and IV with R³ = acetyloxy or benzoyloxy, more preferably with a compound b) selected from the group consisting of PH and IP, most preferably with IP,
using a catalyst not previously used for this reaction, as will be specified hereinafter, in an organic solvent
to obtain a tocol, a tocopherol or α-tocopherol acetate represented by the following formula I with X¹, X² and X³ being independently from each other hydrogen or methyl and R¹ being hydrogen or acetyl, with the proviso that R¹ is only acetyl when X¹, X² and X³ are all methyl. 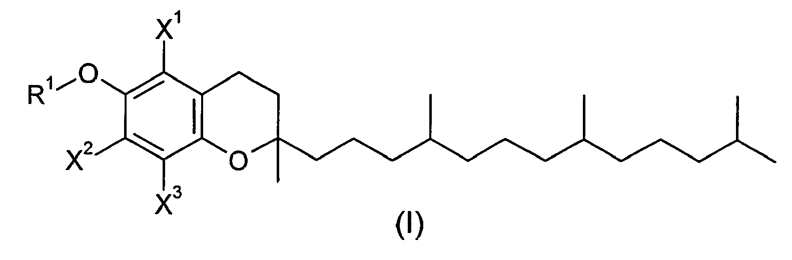 Concerning the substituent R³: Preferred examples for "C₂₋₅-alkanoyloxy" are acetyloxy (H₃C(CO)O), propionyloxy (H₃CCH₂(CO)O) and pivaloyloxy ((H₃C)₃C(CO)O).

The process in accordance with the present invention- a Friedel-Crafts-type alkylation reaction followed by a ring-closure reaction - is characterized by carrying out the reaction in the presence of bismuth trifluoromethanesulphonate, Bi(OSO₂CF₃)₃, as the catalyst in an organic solvent.

While the production of (all-*rac*)-tocols and (all-*rac*)-tocopherols such as (all-*rac*)-α-tocopherol (formula I with X¹ = X² = X³ = CH₃ and R¹ = H) and (all-*rac*)-α-tocopheryl acetate (formula I with X¹ = X² = X³ = methyl and R¹ = acetyl) is preferred, the invention is not limited to the production of that particular isomeric form and other isomeric forms can be obtained by using phytol, isophytol or a derivative thereof as the starting material in the appropriate isomeric form. Thus, e.g. (*RS*,*R*,*R*)-α-tocopherol/(*RS*,*R*,*R*)-α-tocopheryl acetate will be obtained when using (*R*,*R*)-phytol, (R,R,R)-isophytol, (S,R,R)-isophytol or (*RS*,*R*,*R*)-isophytol or an appropriate (iso)phytol derivative. The same applies for the manufacture of the other tocols and tocopherols.

### Manufacture of a tocol or tocopherol, preferably α-tocopherol (formula I with X¹, X² and X³ = CH₃ and R¹ = H)

As one aspect, the present invention is concerned with a novel process for the manufacture of a tocol or tocopherol, preferably TCP (formula I with X¹ = X² = X³ = CH₃ and R¹ = H) by the reaction of a hydroquinone with 0 to 3 methyl groups, preferably by the reaction of TMHQ (formula II with X¹ = X² = X³ = CH₃ and R² = H), with IP, PH or a(n) (iso)phytol derivative represented by the formulae III and IV as defined above in the presence of bismuth trifluoromethanesulphonate, Bi(OSO₂CF₃)₃, as the catalyst in an organic solvent.

### Manufacture of α-tocopheryl acetate (formula I with X¹ = X² = X³ = CH₃ and R¹ = acetyl)

As a further aspect, the present invention is concerned with the manufacture of TCPA (formula I with X¹ = X² = X³ = methyl and R¹ = acetyl) starting from TMHQA (formula II with X¹ = X² = X³ = methyl and R² = acetyl) and IP, PH or a(n) (iso)phytol derivative represented by the formulae III and IV as defined above in the presence of bismuth trifluoromethanesulphonate, Bi(OSO₂CF₃)₃,as the catalyst in an organic solvent.

The starting material TMHQA may be obtained e.g. by selective hydrolysis of 2,3,5-trimethylhydroquinone-diacetate as described in EP 1 239 045. The (iso)phytyl compounds as well as the hydroquinones comprising 0 to 3 methyl groups can be produced by conventional processes known to the person skilled in the art. PH and its derivatives represented by the formula IV can be used as E/Z-mixture as well as in pure E-or pure Z-form. Preferred is the use of PH and its derivatives represented by the formula IV as E/Z-mixture.

In an especially preferred embodiment of the invention TMHQ (formula II with X¹ = X² = X³ = CH₃ and R² = H) is reacted with PH (formula IV with R³ = OH) and/or IP (formula III with R³ = OH), more preferably with IP, to TCP (formula I with X¹ = X² = X³ = CH₃ and R¹ = H).

The catalyst Bi(OSO₂CF₃)₃ can be used in solid form, as well as in solution or as suspension. Preferably the catalyst is dissolved or suspended in the solvent/solvent system, in which the reaction is carried out. The concentration of the solution is not critical. Furthermore, the catalyst tolerates traces of protic solvents such as methanol, ethanol and water. If the reaction is carried out in a biphasic solvent system (see below) the catalyst can be recovered from the polar phase.

Examples of solvents suitable for the reaction of compounds a) and b) to e.g. TCP or TCPA, as appropriate, according to the present invention are polar non-protic organic solvents such as aliphatic and cyclic carbonates, especially ethylene carbonate, propylene carbonate and 1,2-butylene carbonate; aliphatic esters and cyclic esters (lactones), especially ethyl acetate, isopropyl acetate, butyl acetate and γ-butyrolactone; aliphatic and cyclic ketones, especially diethyl ketone, isobutyl methyl ketone, cyclopentanone and isophorone; and mixtures thereof. Especially preferred are ethylene carbonate and/or propylene carbonate.

More preferred are biphasic solvent systems comprising polar and non-polar solvents.

Examples of polar solvents in such biphasic solvent systems are the polar non-protic organic solvents named above. Especially preferred are cyclic carbonates and lactones, especially ethylene carbonate, propylene carbonate and γ-butyrolactone. Most preferred are the cyclic carbonates, especially ethylene carbonate and propylene carbonate and mixtures thereof.

Examples of non-polar solvents in such biphasic solvent systems are aliphatic linear, aliphatic branched or aliphatic cyclic C₅₋₁₅-hydrocarbons, especially linear, branched or cyclic C₅₋₁₅-alkanes. Particularly preferred are linear, branched or cyclic C₆₋₁₀-alkanes, especially preferred are hexane, heptane, octane, cyclohexane and methylcyclohexane or mixtures thereof. The most preferred non-polar solvent is heptane.

The most preferred biphasic solvent systems are mixtures of ethylene carbonate and/or propylene carbonate and hexane, heptane or octane, especially mixtures of ethylene carbonate and heptane, mixtures of propylene carbonate and octane, and mixtures of ethylene carbonate, propylene carbonate and heptane.

The molar ratio of compound a) to compound b) in the reaction mixture conveniently varies from about 1.15 : 1 to about 3 : 1, preferably from about 1.25 : 1 to about 2.2 : 1, more preferably from about 1.5 : 1 to about 2: 1.

The amount of the catalyst Bi(OSO₂CF₃)₃ used is based on the amount of compound a) or b) which is used in the lesser molar amount. Usually the relative amount of Bi(OSO₂CF₃)₃ to the amount of compound a) or b) is from about 0.025 to about 0.1 mol%, preferably from about 0.035 to about 0.09 mol%, more preferably from about 0.05 to about 0.08 mol%. Such catalytic amounts of Bi(OSO₂CF₃)₃ are sufficient to obtain high yields of desired product. In this context the expression "amount of Bi(OSO₂CF₃)₃" is to be understood as referring to the weight of pure bismuth trifluoromethanesulphonate present, even though the catalyst may be impure and/or in the form of an adduct with a solvent.

The amount of the polar solvent used is conveniently from about 0.5 ml to about 2.0 ml, preferably from about 0.6 ml to about 1.75 ml, more preferably from about 0.8 ml to about 1.6 ml, based on 1 mmol of compound b).

In the biphasic solvent systems the amount of polar solvent to the amount of non-polar solvent varies conveniently from about 5 : 1 to about 1 : 10 by volume, preferably from about 3 : 1 to about 1 : 5 by volume, more preferably from about 2 : 1 to about 1 : 1.25 by volume.

It was found that the cyclic carbonate used in the biphasic solvent systems could be recycled several times.

The reaction is conveniently carried out at temperatures from about 80°C to about 160°C, preferably from about 90°C to about 150°C, more preferably from about 100°C to about 145°C.

The pressure under which the reaction is carried out is not critical, but it is conveniently carried out at atmospheric pressure.

Moreover, the process is conveniently carried out under an inert gas atmosphere, preferably gaseous nitrogen or argon.

The actual reaction generally lasts for about 0.5 to 6 hours, preferably about 0.75 to 3 hours, especially about 1 to 2 hours.

The process in accordance with the invention can be carried out batchwise or continuously, and in general operationally in a very simple manner, for example (1) by adding compound b) - as such or dissolved in the non-polar solvent (if the reaction is carried out in a biphasic solvent system) such as mentioned above, preferably as such - portionwise or continuously to a mixture of Bi(OSO₂CF₃)₃, compound a) and the polar solvent/biphasic solvent system.

It is also possible to add, portionwise or continuously, a mixture of Bi(OSO₂CF₃)₃ and compound a) - each dissolved or suspended in the polar solvent - to compound b) or to a mixture of compound a), dissolved or suspended in the polar solvent, and compound b). This can be carried out as follows:
(2) A mixture of Bi(OSO₂CF₃)₃ and compound a), each dissolved or suspended in the polar solvent, is added portionwise or continuously to compound b) - as such or dissolved in the non-polar solvent, if the reaction is carried out in a biphasic solvent system.

If the reaction is carried out with an excess of compound a) (see above) there are two further possibilities:
(3) To a mixture of compound b) - as such, if the reaction is ultimately carried out only in a polar solvent, or dissolved in the non-polar solvent, if the reaction is ultimately carried out in a biphasic solvent system (see above) - and a substantially equimolar amount of compound a), dissolved or suspended in the polar solvent, a mixture of Bi(OSO₂CF₃)₃ and compound a), each dissolved or suspended in the polar solvent, is added. The added amount of compound a) is here the substantial excess amount of compound a) over the employed amount of compound b).
(4) To a mixture of compound b) - as such, if the reaction is ultimately carried out only in a polar solvent, or dissolved in the non-polar solvent, if the reaction is ultimately carried out in a biphasic solvent system (see above) - and compound a), dissolved or suspended in the polar solvent, whereby the amount of compound a) is the substantial excess amount of compound a) over the employed amount of compound b), is added a mixture of Bi(OSO₂CF₃)₃ and compound a), each dissolved or suspended in the polar solvent. The added amount of compound a) is here substantially the equimolar amount relative to the employed amount of compound b).

Furthermore, if the reaction is carried out in a biphasic solvent system, it is also possible to add, portionwise or continuously, compound a) - dissolved or suspended in the polar solvent - or a mixture of Bi(OSO₂CF₃)₃ and compound a) - each dissolved or suspended in the polar solvent - to a mixture of compound b), dissolved in the non-polar solvent, and Bi(OSO₂CF₃)₃ and compound a) - each dissolved or suspended in the polar solvent. This can be carried out in three different ways:
(5) To a mixture of compound b), dissolved in the non-polar solvent, and Bi(OSO₂CF₃)₃ and compound a), each dissolved or suspended in the polar solvent, whereby the amount of compound a) is the substantial excess amount of compound a) over the employed amount of compound b), is added a solution or suspension of compound a) in the polar solvent. The added amount of compound a) is here substantially the equimolar amount relative to the employed amount of compound b).
(6) To a mixture of compound b), dissolved in the non-polar solvent, and Bi(OSO₂CF₃)₃ and a substantially equimolar amount of compound a), each dissolved or suspended in the polar solvent, is added a solution or suspension of compound a) in the polar solvent. The added amount of compound a) is here the substantial excess amount of compound a) over the employed amount of compound b).
(7) To a mixture of compound b), dissolved in the non-polar solvent, and Bi(OSO₂CF₃)₃ and compound a), each dissolved or suspended in the polar solvent, whereby the amounts of Bi(OSO₂CF₃)₃ and compound a) in this mixture are less than their total amounts used in the reaction, are added further amounts of Bi(OSO₂CF₃)₃ and compound a), each dissolved or suspended in the polar solvent. Preferably the polar solution containing Bi(OSO₂CF₃)₃ and compound a), with which the reaction is started, and the added polar solution containing Bi(OSO₂CF₃)₃ and compound a) have the same concentration.

Preferred are methods 1 and 4, most preferred is method 1.

The rate of addition of the one component to the other is not critical. Conveniently, the component to be added is added continuously over a period from about 20 to about 90 minutes, preferably from about 25 to about 75 minutes, more preferably from about 30 to about 60 minutes, independently of the scale in which the process is performed.

If compound b) is dissolved in a non-polar solvent such as mentioned above, the resulting concentration of compound b) in the solution is conveniently from about 5 to about 35% by weight, preferably from about 10 to about 30% by weight, more preferably from about 15 to about 25% by weight, based on the total weight of the solution.

The concentration of compound a), dissolved or suspended in the polar solvent (with or without Bi(OSO₂CF₃)₃), usually ranges from about 5 to about 25% by weight, preferably from about 5 to about 20% by weight, more preferably from about 10 to about 17% by weight, (in each case) based on the total weight of the solution.

After completion of the addition of compound b) (in the non-polar solvent) or compound a) in the polar solvent or the mixture of Bi(OSO₂CF₃)₃ and compound a) in the polar solvent and an appropriate subsequent reaction period the isolation of the product and its purification if required, can be effected by procedures conventionally used in organic chemistry.

The present invention provides e.g. a highly selective and high-yield manufacture of TCP.

Further advantages of the use of the catalyst in the process according to the present invention are the avoidance of corrosion, the avoidance of waste water contamination with chlorinated by-products, the enabled ready isolation of the produced tocols and tocopherols such as (all-*rac*)-TCP from the mixture after reaction.

### Process for the manufacture of formulations of α-tocopherol and its esters

The TCP obtained by the process of the present invention or its esters obtained therefrom according to standard methods can further be formulated by any method known to the person skilled in the art, e.g. as those disclosed in WO 96/01103**,** WO 98/15195 and US 6,030,645**.**

The following examples illustrate the invention in more detail, but are not intended to limit its scope in any way.

### Examples

### General Remarks

TMHQ (98%, Fluka, Buchs) was used without further purification. IP (96.3%, Teranol, Lalden) was added with a Metrohm 665 Dosimat. The phytyl acetate (98 %, E/Z = 2.5/1) and phytyl benzoate (96%, E/Z = 2.4/1) were dissolved in heptane and added with a dropping funnel. The crude products were analyzed by gas chromatography (GC).

Bi(OSO₂CF₃)₃ was synthesized from Bi₂O₃ as described in S. Répichet, Tetrahedron Letters, 2002, 43, 993-995. The used catalyst contained 6.37 % water and 1.25 % ethanol - determined by the elementar analysis according to Karl Fischer.

The amount of catalyst was calculated on basis of the pure catalyst and based on the amount of IP used.

All reactions were carried out under argon.

### Example A: Preparation of (all-rac)-TCP in polar organic solvents

In a 200 ml four-necked flask equipped with a mechanical stirrer, a water separator and a reflux condenser 5.04 g (33 mmol) of TMHQ and given amounts of solvent (γ-butyrolactone or diethylketone) and Bi(OSO₂CF₃)₃ (see table 1 below) were heated up under argon atmosphere to reflux temperature (oil bath 140 to 145°C). 11.9 ml (33 mmol) of IP were added at a rate of 0.6 ml/minute. The reaction mixture was heated under reflux for 30 to 60 minutes after completion of the addition of the IP. The reaction mixture was cooled down and evaporated under reduced pressure. A viscous oil was obtained. For the yield of (all-*rac*)-TCP see table 1.

### Example B: Preparation of (all-rac)-TCP in cyclic carbonates

In a four-necked flask equipped with a mechanical stirrer, a water separator and a reflux condenser 49.5 mmol of TMHQ, 0.036 mmol of Bi(OSO₂CF₃)₃ and a given amount of the cyclic carbonate(s) (amount and type see table 1) were heated up under an argon atmosphere to 103-105°C (oil bath: 145°C). 11.9 ml (33 mmol) of IP were added at a rate of 0.6 ml per minute during 20 minutes. The reaction mixture was heated additionally for 30 to 60 minutes after completion of the addition of the IP. The reaction mixture was cooled down to 80°C and 50 ml of heptane were added to the carbonate phase. The mixture was stirred for additional 10 minutes and the phases separated. The heptane phase was evaporated under reduced pressure. A viscous oil was obtained. For the yield of (all-*rac*)-TCP see table 1.

Table 1: Reaction of TMHQ and IP to (all-*rac*)-TCP in selected polar solvents. The amount of Bi(OSO₂CF₃)₃ and the yield are based on IP, the molar ratio of TMHQ to IP was 1 to 1 in all cases. As can be seen from table 2 best yields are obtained in biphasic solvent systems.

| Bi(OSO₂CF₃)₃ [mol%] - based on IP | Solvent | yield TCP [%] |
|---|---|---|
| 0.1 | diethyl ketone (50 ml) | 65.5 |
| 0.1 | γ-butyrolactone (50 ml) | 61.2 |
| 0.11 | ethylene carbonate (40 g) | 91.3 |
| 0.11 | propylene carbonate (30 g) | 91.2 |
| 0.11 | ethylene carbonate/propylene carbonate (15 g/15 g) | 90.5 |

### Example C: Preparation of (all-rac)-TCP in biphasic solvent systems

In a 200 ml four-necked flask equipped with a mechanical stirrer, a water separator and a reflux condenser 7.53 g (50 mmol) of TMHQ and given amounts of Bi(OSO₂CF₃)₃ and the biphasic solvent system (amount see table 2 below) were heated up under an argon atmosphere to reflux (oil bath: 140°C). 11.9 ml (33 mmol) of IP were added at a rate of 0.6 ml/minute. Approximately 0.9 ml of water were collected after complete addition of the isophytol. The heptane was distilled off within approximately 20 minutes. Afterwards the reaction mixture was heated for 30 minutes at 125 to 130°C. The reaction mixture was cooled down to 80°C. 50 ml of heptane were added to the carbonate phase. The reaction mixture was stirred for an additional 10 minutes at 50°C. The heptane layer was separated and evaporated under reduced pressure. A viscous oil was obtained. For the yield of (all-*rac*)-TCP see table 2 below.
The carbonate phase can be reused.

### Example D: Different order of the addition of the components

In a 200 ml four-necked flask equipped with a mechanical stirrer, a water separator and a reflux condenser 7.53 g (50 mmol) of TMHQ, 11.9 ml (33 mmol) of IP and 50 ml of heptane were heated up under an argon atmosphere to 103-105°C. 0.0366 mmol of Bi(OSO₂CF₃)₃ dissolved in 30 g of propylene carbonate were added during 2 hours.
During the reaction water was continuously removed. Afterwards the reaction mixture was heated for 60 minutes at 103°C. The reaction mixture was cooled down to 80°C, the phases were separated and the heptane phase concentrated under reduced pressure. A viscous oil was obtained. The yield of (all-*rac*)-TCP was 92.5% - based on IP.

Table 2: Influence of the amount of Bi(OSO₂CF₃)₃ on the reaction of TMHQ and IP to (all-*rac*)-TCP in biphasic solvent systems. The amount of Bi(OSO₂CF₃)₃ and the yield are based on IP, the molar ratio of TMHQ to IP was 1.5 to 1 in ethylene carbonate/heptane, in all other cases 1 to 1. ¹⁾ reaction time = 360 minutes (all other cases 30 minutes)

| Bi(OSO₂CF₃)₃ [mol%] - based on IP | Solvent | yield TCP [%] |
|---|---|---|
| 0.11 | heptane/propylene carbonate (30 g/50 ml) (example D) | 92.5 |
| 0.1 | propylene carbonate/heptane (30 g/50 ml) | 95.3 |
| 0.1 | γ-butyrolactone/heptane (40 g/50 ml) | 86.9 |
| 0.1 | ethylene carbonate/heptane (40 g/50 ml) | 95.7 |
| 0.075 | ethylene carbonate/heptane (40 g/50 ml) | 94.1 |
| 0.05 | ethylene carbonate/heptane (40 g/50 ml) | 96.8 |
| 0.1 | ethylene carbonate/propylene carbonate/heptane (15 g/15 g/50 ml) | 98.1 |
| 0.075 | ethylene carbonate/propylene carbonate/heptane (15 g/15 g/50 ml) | 98.0 |
| 0.05 | ethylene carbonate/propylene carbonate/heptane (15 g/15 g/50 ml) | 98.3 |
| 0.025 | ethylene carbonate/propylene carbonate/heptane (15 g/15 g/50 ml) | 61.4 |
| 0.025 | ethylene carbonate/propylene carbonate/heptane (15 g/15 g/50 ml) | 85.5¹⁾ |

### Example E: Synthesis of (all-rac)-TCPA in a biphasic solvent system

In a 200 ml four-necked flask equipped with a mechanical stirrer, a thermometer, a water separator and a reflux condenser 9.7 g (49.5 mmol) of TMHQA, 40 g of ethylene carbonate, given amounts of the catalyst Bi(OSO₂CF₃)₃ (see table 3 below) and 50 ml of heptane were filled in. The reaction mixture was heated up under an argon atmosphere to reflux (oil bath: 140 to 145°C). When heptane (boiling temperature: 99.4°C) refluxed, 11.9 ml (33 mmol) IP was added dropwise at a rate of 0.595 ml/minute. The heptane was distilled off to remove the water formed during the reaction and the resulting mixture heated at 120°C for 30 minutes. Then the mixture was cooled down to 80°C, 50 ml of heptane were added, and the resulting mixture was stirred for additional 10 minutes at 50°C. After separation of the phases the heptane layer was evaporated under reduced pressure starting at 95 mbar and going continuously to 10 mbar within 1 hour. A viscous oil was obtained and analyzed by GC. The results are summarized in table 3.

**Table 3: Reaction of IP and TMHQA to (all-rac)-TCPA in the presence of Bi(OSO₂CF₃)₃ as catalyst in a through process. The yields are based on IP.**

| Bi(OSO₂CF₃)₃ [mol%] - based on IP | yield TCPA [%] | yield TCP [%] | yield "phytadienes"²⁾ [%] |
|---|---|---|---|
| 0.1 | 78.5 | 12.0 | 4.0 |
| 0.075 | 83.1 | 6.9 | 4.7 |
| 0.051 | 82.2 | 7.0 | 4.1 |

| | | | |
|---|---|---|---|
| ²⁾ "Phytadienes" are the by-products resulting from compound b) and defined as the sum of compounds detected in the range of retention time t_{R} from 6.7 to 8.5 minutes in GC. | | | |

### Example F: Preparation of (all-rac) TCP starting from TMHQ and E/Z-phytyl acetate in a biphasic solvent system

In a 50-ml four-necked flask equipped with a mechanical stirrer and a reflux condenser, 1.14 g (7.5 mmol) of TMHQ, 4.1 mg (0.1 mol%) of Bi(OSO₂CF₃)₃, 6 g of ethylene carbonate, and 5 ml of heptane were heated up under argon atmosphere to reflux (oil bath 145°C, stirring 300 rotations per minute). 1.7 g (4.92 mmol) of E/Z (all-*rac*)-phytyl acetate were dissolved in 5 ml of heptane and added through a dropping funnel within 50 minutes. The dropping funnel was washed with 2 ml of heptane. The heptane was distilled off within approximately 10 minutes. Afterwards the reaction mixture was heated for 2 hours and 15 minutes at 125 to 130°C. The reaction mixture was cooled to 80°C and 10 ml of heptane were added to the mixture. The reaction mixture was stirred for additional 10 minutes at 50°C. The heptane layer was separated and evaporated under reduced pressure starting at 100 mbar and going continuously to 10 mbar within 1 hour. Yield: 1.79 g of (all-*rac*)-TCP (viscous oil), 84.5% based on phytyl acetate.

### Example G: Preparation of (all-rac)-TCP starting from TMHO and E/Z-phytyl benzoate in a biphasic solvent system

In a 50-ml four-necked flask equipped with a mechanical stirrer and a reflux condenser, 0.95 g (6.2 mmol) of TMHQ, 3.4 mg (0.1 mol%) of Bi(OSO₂CF₃)₃, 5 g of ethylene carbonate, and 5 ml of heptane were heated up under argon atmosphere to reflux (oil bath 145°C, stirring 300 rotations per minute). 1.72 g (4.12 mmol) of E/Z (all-*rac*)-phytyl benzoate were dissolved in 5 ml of heptane and added through a dropping funnel within 50 minutes. The dropping funnel was washed with 2 ml of heptane. The heptane was distilled off within approximately 10 minutes. Afterwards the reaction mixture was heated for 2 hours and 15 minutes at 125 to 130°C. The reaction mixture was cooled to 80°C and 10 ml of heptane were added to the mixture. The reaction mixture was stirred for additional 10 minutes at 50°C. The heptane layer was separated and evaporated under reduced pressure starting at 100 mbar and going continuously to 10 mbar within 1 hour. Yield: 1.75 g of (all-*rac*)-TCP (viscous oil), 98.6 % based on phytyl benzoate.

## Claims

1. A process for the manufacture of a compound of the following formula I with X¹, X² and X³ being independently from each other hydrogen or methyl and R¹ being hydrogen or acetyl, with the proviso that R¹ is only acetyl when X¹, X² and X³ are all methyl, 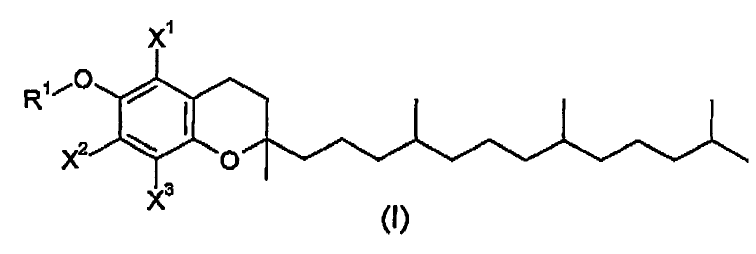 by the catalyzed reaction of
a compound a) of the formula II with X¹, X² and X³ being independently from each other hydrogen or methyl and R² being hydrogen or acetyl with the proviso that R² is only acetyl when X¹, X² and X³ are all methyl, 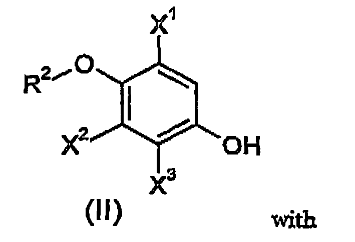 a compound b) selected from the group consisting of phytol, isophytol and (iso)-phytol derivatives represented by the following formulae III and IV with R³ = C₂₋₅-alkanoyloxy, benzoyloxy, mesyloxy, benzenesulfonyloxy or tosyloxy 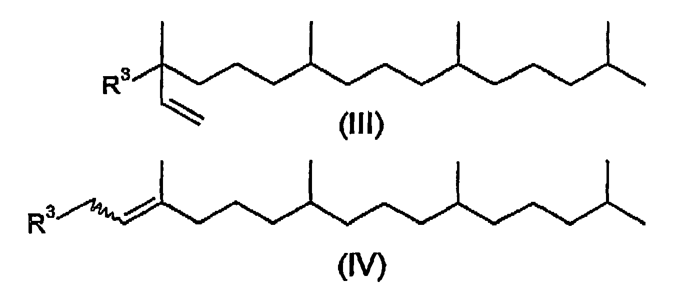 **characterized in that** the reaction is carried out in the presence of bismuth trifluoromethanesulphonate, Bi(OSO₂CF₃)₃, as the catalyst in an organic solvent.

2. The process as claimed in claim 1 wherein the C₂₋₅-alkanoyloxy is selected from the group consisting of acetyloxy, propionyloxy and pivaloyloxy.

3. The process as claimed in claim 1 wherein 2,3,5-trimethylhydroquinone (formula II with X¹ = X² = X³ = CH₃ and R² = H) or its acetate (formula II with X¹ *=* X² = X³ = CH₃ and R² = acetyl) is reacted with a compound b) selected from the group consisting of phytol, isophytol and (iso)phytol derivatives represented by the following formulae III and IV with R³ = acetyloxy or benzoyloxy, to α-tocopherol (formula I with X¹ = X² = X³ = CH₃ and R¹ = H) or α-tocopheryl acetate (formula I with X¹ = X² = X³ = CH₃ and R¹ = acetyl).

4. The process as claimed in claim 1 wherein 2,3,5-trimethylhydroquinone or 2,3,6-trimethylhydroquinone-1-acetate (compound a)) is reacted with phytol and/or isophytol, preferably with isophytol, to α-tocopherol or α-tocopheryl acetate.

5. The process as claimed in one or more of the proceeding claims wherein the organic solvent is a polar solvent selected from the group consisting of aliphatic and cyclic carbonates, aliphatic esters and cyclic esters, aliphatic and cyclic ketones, and mixtures thereof.

6. The process as claimed in claim 5, wherein the organic solvent is ethylene carbonate and/or propylene carbonate.

7. The process as claimed in claims 1 to 4, wherein the organic solvent is a biphasic solvent system of a polar solvent and a non-polar solvent, the polar solvent being at least a cyclic carbonate, and the non-polar solvent being at least an aliphatic linear, aliphatic branched or aliphatic cyclic C₅₋₁₅-hydrocarbon, preferably a linear, branched or cyclic C₅₋₁₅-alkane.

8. The process as claimed in claim 7 wherein the polar solvent is ethylene carbonate or propylene carbonate or a mixture thereof, and the non-polar solvent is hexane, heptane, octane, cyclohexane, methylcyclohexane or a mixture thereof, preferably heptane.

9. The process as claimed in claims 7 or 8, wherein the volume ratio of the polar solvent to the non-polar solvent in the biphasic solvent system is in the range from about 5 : 1 to about 1 : 10, preferably from about 3 : 1 to about 1 : 5, especially from about 2 : 1 to about 1 : 1.25.

10. The process as claimed in claims 5 to 9, wherein from about 0.5 ml to about 2.0 ml, preferably from about 0.6 ml to about 1.75 ml, more preferably from about 0.8 ml to about 1.6 ml of a polar organic solvent are used per mmol of compound b).

11. The process as claimed in one or more of the proceeding claims wherein the relative amount of the catalyst Bi(OSO₂CF₃)₃ to the amount of compound a) or compound b), whichever is in the lesser molar amount is from about 0.025 mol% to about 0.1 mol%, preferably from about 0.035 mol% to about 0.09 mol%, more preferably from about 0.05 to about 0.08 mol%.

12. The process according to one or more of the proceeding claims, wherein the molar ratio of compound a) to compound b) present in the reaction mixture is from about 1.15 : 1 to about 3 : 1, preferably from about 1.25 : 1 to about 2.2 : 1, more preferably from about 1.5 : 1 to about 2 : 1.

13. The process according to claims 5 to 12, wherein compound b) as such or in solution is added portionwise or continuously to a mixture of Bi(OSO₂CF₃)₃, compound a) and the polar solvent/biphasic solvent system.

14. The process as claimed in one or more of the proceeding claims wherein the reaction is carried out at temperatures from about 80°C to about 160°C, preferably from about 90°C to about 150°C, especially from about 100°C to about 145°C.

15. A process for the manufacture of formulations of α-tocopherol and α-tocopherol acetate wherein α-tocopherol and its acetate obtained by a process as claimed in one or more of the proceeding claims is used.

## Patentansprüche

1. Verfahren zur Herstellung einer Verbindung der folgenden Formel I, wobei X¹, X² und X³ unabhängig voneinander für Wasserstoff oder Methyl stehen und R¹ für Wasserstoff oder Acetyl steht, mit der Maßgabe, daß R¹ nur dann für Acetyl steht, wenn X¹, X² und X³ alle für Methyl stehen, 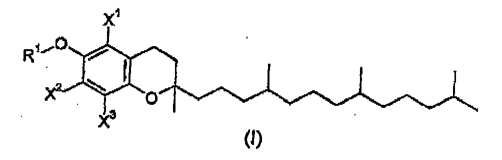 durch die katalysierte Reaktion einer Verbindung a) der Formel II, wobei X¹, X² und X³ unabhängig voneinander für Wasserstoff oder Methyl stehen und R² für Wasserstoff oder Acetyl steht, mit der Maßgabe, daß R² nur dann für Acetyl steht, wenn X¹, X² und X³ alle für Methyl stehen, 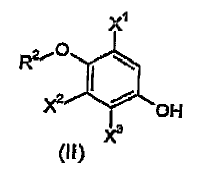 mit einer Verbindung b) aus der Gruppe bestehend aus Phytol, Isophytol und (Iso)phytolderivaten der folgenden Formeln III und IV mit R³ = C₂₋₅-Alkanoyloxy, Benzoyloxy, Mesyloxy, Benzolsulfonyloxy oder Tosyloxy, 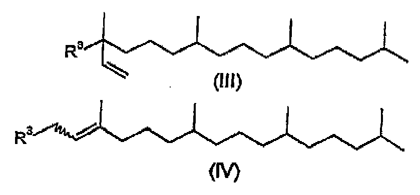 **dadurch gekennzeichnet, daß** die Reaktion in Gegenwart von Bismuttrifluormethansulfonat, Bi(OSO₂CF₃)₃, als Katalysator in einem organischen Lösungsmittel durchgeführt wird.

2. Verfahren nach Anspruch 1, bei dem das C₂₋₅-Alkanoyloxy aus der Gruppe bestehend aus Acetyloxy, Propionyloxy und Pivaloyloxy ausgewählt wird.

3. Verfahren nach Anspruch 1, bei dem 2,3,5-Trimethylhydrochinon (Formel II mit X¹ = X² = X³ = CH₃ und R² = H) oder sein Acetat (Formel II mit X¹ = X² = X³ = CH₃ und R² = Acetyl) mit einer Verbindung b) aus der Gruppe bestehend aus Phytol, Isophytol und (Iso)phytolderivaten der folgenden Formeln III und IV mit R³ = Acetyloxy oder Benzoyloxy zu α-Tocopherol (Formel I mit X¹ = X² = X³ = CH₃ und R¹ = = H) oder α-Tocopherylacetat (Formel I mit X¹ = X² = X³ = CH₃ und R¹ = Acetyl) umgesetzt wird.

4. Verfahren nach Anspruch 1, bei dem 2,3,5-Trimethylhydrochinon oder 2,3,6-Trimethylhydrochinon-1-acetat (Verbindung a)) mit Phytol und/oder Isophytol, vorzugsweise mit Isophytol, zu α-Tocopherol oder α-Tocopherylacetat umgesetzt wird.

5. Verfahren nach einem oder mehreren der vorhergehenden Ansprüche, bei dem es sich bei dem organischen Lösungsmittel um ein polares Lösungsmittel aus der Gruppe bestehend aus aliphatischen und cyclischen Carbonaten, aliphatisschen Estern und cyclischen Estern, aliphatischen und cyclischen Ketonen und Mischungen davon handelt.

6. Verfahren nach Anspruch 5, bei dem es sich bei dem organischen Lösungsmittel um Ethylencarbonat und/oder Propylencarbonat handelt.

7. Verfahren nach den Ansprüchen 1 bis 4, bei dem es sich bei dem organischen Lösungsmittel um ein zweiphasiges Lösungsmittelsystem aus einem polaren Lösungsmittel und einem unpolaren Lösungsmittel handelt, wobei das polare Lösungsmittel mindestens ein cyclisches Carbonat ist und das unpolare Lösungsmittel mindestens ein aliphatischer linearer, aliphatischer verzweigter oder aliphatischer cyclischer C₅₋₁₅-Kohlenwasserstoff, vorzugsweise ein lineares, verzweigtes oder cyclisches C₅₋₁₅-Alkan, ist.

8. Verfahren nach Anspruch 7, bei dem es sich bei dem polaren Lösungsmittel um Ethylencarbonat oder Propylencarbonat oder eine Mischung davon handelt und es sich bei dem unpolaren Lösungsmittel um Hexan, Heptan, Octan, Cyclohexan, Methylcyclohexan oder eine Mischung davon, vorzugsweise Heptan, handelt.

9. Verfahren nach den Ansprüchen 7 oder 8, bei dem das Volumenverhältnis des polaren Lösungsmittels zu dem unpolaren Lösungsmittel in dem zweiphasigen Lösungsmittelsystem im Bereich von etwa 5:1 bis etwa 1:10, vorzugsweise etwa 3:1 bis etwa 1:5, insbesondere etwa 2:1 bis etwa 1:1,25, liegt.

10. Verfahren nach den Ansprüchen 5 bis 9, bei dem etwa 0,5 ml bis etwa 2,0 ml, vorzugsweise etwa 0,6 ml bis etwa 1,75 ml, besonders bevorzugt etwa 0,8 ml bis etwa 1,6 ml, eines polaren organischen Lösungsmittels pro mmol Verbindung b) verwendet werden.

11. Verfahren nach einem oder mehreren der vorhergehenden Ansprüche, bei dem die relative Menge des Katalysators Bi(OSO₂CF₃)₃ zur Menge an Verbindung a) oder Verbindung b), welche auch immer in der geringeren molaren Menge vorliegt, etwa 0,025 Mol-% bis etwa 0,1 Mol-%, vorzugsweise etwa 0,035 Mol-% bis etwa 0,09 Mol-%, besonders bevorzugt etwa 0,05 bis etwa 0,08 Mol-%, beträgt.

12. Verfahren nach einem oder mehreren der vorhergehenden Ansprüche, bei dem das Molverhältnis von Verbindung a) zu Verbindung b) in der Reaktionsmischung etwa 1,15:1 bis etwa 3:1, vorzugsweise etwa 1,25:1 bis etwa 2,2:1, besonders bevorzugt etwa 1,5:1 bis etwa 2:1, beträgt.

13. Verfahren nach den Ansprüchen 5 bis 12, bei dem Verbindung b) als solche oder in Lösung portionsweise oder kontinuierlich zu einer Mischung von Bi(OSO₂CF₃)₃, Verbindung a) und dem polaren Lösungsmittel/zweiphasigen Lösungsmittelsystem gegeben wird.

14. Verfahren nach einem oder mehreren der vorhergehenden Ansprüche, bei dem die Reaktion bei Temperaturen von etwa 80°C bis etwa 160°C, vorzugsweise etwa 90°C bis etwa 150°C, insbesondere etwa 100°C bis etwa 145°C, durchgeführt wird.

15. Verfahren zur Herstellung von Formulierungen von α-Tocopherol und α-Tocopherolacetat, bei dem α-Tocopherol und sein Acetat, erhalten durch ein Verfahren nach einem oder mehreren der vorhergehenden Ansprüche, verwendet wird.

## Revendications

1. Procédé de fabrication d'un composé de la formule I suivantes, X¹, X² et X³ étant, indépendamment les uns des autres, hydrogène ou méthyle et R¹ étant hydrogène ou acétyle, à condition que R¹ ne soit qu'acétyle lorsque X¹, X² et X³ sont tous méthyle, 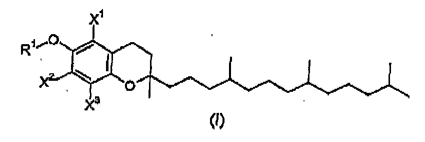 par la réaction catalysée
d'un composé a) de formule II, X¹, X² et X³ étant, indépendamment les uns des autres, hydrogène ou méthyle et R² étant hydrogène ou acétyle, à condition que R² ne soit qu'acétyle lorsque X¹, X² et X³ sont tous méthyle, 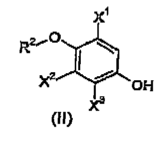 avec
un composé b) choisi du groupe constitué de phytol, d'isophytol et de dérivés d'(iso)phytol représentés par les formules III et IV suivantes avec R³ = C₂₋₅-alcanoyloxy, benzoyloxy, mésyloxy, benzènesulfonyloxy ou tosyloxy 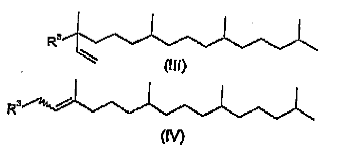 **caractérisé en ce que** la réaction est effectuée en présence de trifluorométhanesulfonate de bismuth, Bi(OSO₂CF₃)₃, en tant que catalyseur dans un solvant organique.

2. Procédé selon la revendication 1, **caractérisé en ce que** C₂₋₅-alcanoyloxy est choisi dans le groupe constitué d'acétyloxy, propionyloxy et pivaloyloxy.

3. Procédé selon la revendication 1, **caractérisé en ce que** de la 2,3,5-triméthylhydroquinone (formule II avec X¹ = X² = X³ = CH₃ et R² = H) ou son acétate (formule II avec X¹ = X² = X³ = CH₃ et R² = acétyle) est mis en réaction avec un composé b) choisi dans le groupe constitué de phytol, d'isophytol et de dérivés d'(iso)phytol représentés par les formules III et IV suivantes avec R³ = acétyloxy ou benzoyloxy, en α-tocophérol (formule I avec X¹ = X² = X³ = CH₃ et R¹ = H) ou acétate d'α-tocophérol (formule I avec X¹ = X² = X³ = CH₃ et R¹ = acétyle).

4. Procédé selon la revendication 1, **caractérisé en ce que** de la 2,3,5-triméthylhydroquinone ou du 2,3,6-triméthylhydroquinone-1-acétate (composé a)) est mis en réaction avec du phytol et/ou de l'isophytol, de préférence avec de l'isophytol, en α-tocophérol ou en acétate d'α-tocophérol.

5. Procédé selon une ou plusieurs des revendications précédentes, **caractérisé en ce que** le solvant organique est un solvant polaire choisi dans le groupe constitué de carbonates aliphatiques et cycliques, d'esters aliphatiques et d'esters cycliques, de cétones aliphatiques et cycliques, et des mélanges de ceux-ci.

6. Procédé selon la revendication 5, **caractérisé en ce que** le solvant organique est le carbonate d'éthylène et/ou le carbonate de propylène.

7. Procédé selon les revendications 1 à 4, **caractérisé en ce que** le solvant organique est un système de solvant biphasique d'un solvant polaire et d'un solvant non polaire, le solvant polaire étant au moins un carbonate cyclique, et le solvant non polaire étant au moins un hydrocarbure en C₅₋₁₅ aliphatique linéaire, aliphatique ramifié ou aliphatique cyclique, de préférence un alcane en C₅₋₁₅ linéaire, ramifié ou cyclique.

8. Procédé selon la revendication 7, **caractérisé en ce que** le solvant polaire est le carbonate d'éthylène ou le carbonate de propylène ou un mélange de ceux-ci, et le solvant non polaire est l'hexane, l'heptane, l'octane, le cyclohexane, le méthylcyclohexane ou un mélange de ceux-ci, de préférence l'heptane.

9. Procédé selon la revendication 7 ou 8, **caractérisé en ce que** le rapport en volume du solvant polaire sur le solvant non polaire dans le système de solvant biphasique est dans la gamme d'environ 5:1 à environ 1:10, de préférence d'environ 3:1 à environ 1:5, notamment d'environ 2:1 à environ 1:1,25.

10. Procédé selon les revendications 5 à 9, **caractérisé en ce qu'**on utilise d'environ 0,5 ml à environ 2,0 ml, de préférence d'environ 0,6 ml à environ 1,75 ml, plus préférablement d'environ 0,8 ml à environ 1,6 ml d'un solvant organique polaire par mmole de composé b).

11. Procédé selon une ou plusieurs des revendications précédentes, **caractérisé en ce que** la quantité relative du catalyseur Bi(OSO₂CF₃)₃ par rapport à la quantité de composé a) ou de composé b), quel que soit celui qui est en quantité molaire inférieure, est d'environ 0,025 % molaire à environ 0,1 % molaire, de préférence d'environ 0,035 % molaire à environ 0,09 % molaire, plus préférablement d'environ 0,05 à environ 0,08 % molaire.

12. Procédé selon une ou plusieurs des revendications précédentes, **caractérisé en ce que** le rapport molaire du composé a) sur le composé b) présent dans le mélange réactionnel est d'environ 1,15:1 à environ 3:1, de préférence d'environ 1,25:1 à environ 2,2:1, plus préférablement d'environ 1,5:1 à environ 2:1.

13. Procédé selon les revendications 5 à 12, **caractérisé en ce que** le composé b) tel quel ou en solution est ajouté par portions ou en continu à un mélange constitué de Bi(OSO₂CF₃)₃, du composé a) et du système de solvant polaire/solvant biphasique.

14. Procédé selon une ou plusieurs des revendications précédentes, **caractérisé en ce que** la réaction est effectuée à des températures d'environ 80°C à environ 160°C, de préférence d'environ 90°C à environ 150°C, notamment d'environ 100°C à environ 145°C.

15. Procédé de fabrication de formulations d'α-tocophérol et d'acétate d'α-tocophérol, **caractérisé en ce qu'**on utilise l'α-tocophérol et son acétate obtenu par un procédé selon une ou plusieurs des revendications précédentes.
